Europäisches Patentamt

European Patent Office

Office européen des brevets

(11) Publication number: **0 384 960**
**A2**

## (12) EUROPEAN PATENT APPLICATION

(21) Application number: 89119077.9

(51) Int. Cl.⁵: **A61B 5/02**

(22) Date of filing: 13.10.89

(30) Priority: **02.03.89 JP 50689/89**
**02.03.89 JP 50690/89**

(43) Date of publication of application:
**05.09.90 Bulletin 90/36**

(84) Designated Contracting States:
**DE GB**

(71) Applicant: **FUKUDA DENSHI CO., LTD.**
**39-4, Hongo 3-chome Bunkyo-ku**
**Tokyo 113(JP)**

(72) Inventor: **Ishii, Shoji**
**c/o Fukuda Denshi Co. Ltd. Hongo enterprise**
**place**
**2-35-8 Hongo Bunkyo-ku Tokyo(JP)**
Inventor: **Yukimitsu, Yasuji**
**c/o Fukuda Denshi Co. Ltd. Hongo enterprise**
**place**
**2-35-8 Hongo Bunkyo-ku Tokyo(JP)**

(74) Representative: **Behn, Klaus, Dipl.-Ing.**
**Patentanwalt Lindenberg 34**
**D-8134 Pöcking bei München(DE)**

(54) Method for setting alarm and wave indicating sensitivity.

(57) A method for setting alarm comprising, e present time value, the upper limit and lower li·· ⌐ of the living body signal being indicated, the operation of setting the threshold of the upper limit or lower limit carried out by pushing keies indicated and a method for setting wave indication sensitivity comprising, a plurality of keies for setting sensitivity indicated, wave indicating sensitivity being setted by pushing anyine of said indicated keies.

# Fig. 1

## METHOD FOR SETTING ALARM AND WAVE INDICATING SENSITIVITY

BACKGROUND OF THE INVENTION

1. Field of the Invention

The present invention relates to method for setting alarm and wave indicating sensitivity. More particularly, it relates to method for setting alarm and wave indicating sensitivity, which are applied to an apparatus for monitorring the patient.

2. Description of the Related Art

Generally, in an apparatus for monitorring the patient, composed of ECG central monitor etc, a method for settingalarm and a method for setting wave indicating sensitivity are used respectively.

The method for setting alram is as follows.

That is to say, when the present time value of the living body signal, for example heart rate, of the patient is more than the fixed upper limit or the fixed lower limit, an alarm is adopted to be sounded.

In the prior art, the operation of setting previously the above upper and lower limits is carried out by means of volume.

Next, the method for setting wave indicating sensitivity as follows.

That is to say, when the wave of the living body signal of the patient, for example electrocardiogram, is indicated, the sensitivity thereof is adopted to be setted.

Ordinally, in an electrocardiograph, the index of the recorder is adjusted to shake by 10 mm, with respect to 1 mV of electromotive force of the heart, in which case wave indicating sensitivity is defined "1".

That is to say, when 10mm along to the axis of ordinates corresponds to 1mV, the wave indicating sensitivity is 1.

Therefore, when 5mm along to the axis of ordinates corresponds to 1mV, the wave indicating sensitivity is 1/2.

More over, when 20 mm along to the axis of ordinates corresponds to 1mV, the weve indicating sensitivity is 2.

In the prior art, the above sensitivity is setted, by pushing several times a key for setting sensitivity.

The defects of the aforementioned conventional methed are as follows.

With respect to method for setting alarm, at first, since volume is used, the place thereof is occupied.

Hence, the whole apparatus is bigger.

Next, since the present time value of the living body of the patient is not indicated, it is impossible to set the upper limit or lower limit by looking at the indication of the above present time value.

There by, it takes a great deal of trouble to set the upper limit or lower limit.

Moreover, owing to volume, the expense of the apparatus is great.

With respect to method for setting wave indicating sensitivity, it takes a great deal of trouble to set sensitivity.

Thet is to say, it is necessary to do several times the operation of pushing repeatedly a key for setting sensitivity, as aforementioned.

SUMMARY OF THE INVENTION

An object of the present invention is to provide a method for setting alarm, which setting operation is done easily, which place is not occupied, and which cost is low.

Another object of the present invention is to do easily the operation of setting wave indicating sensitivity.

The above-mentioned first object can be achieved by a method for setting alarm comprising, the present time value, the upper limit and lower limit of the living body signal being indicated, the operation of setting the threshold of the upper limit or lower limit carried out by pushing keies indicated.

The above-mentioned second object can be achieved by a method for setting wave indicating sensitivity comprising, a plurality of keies for setting sensitivity indicated, wave indicating sensitivity being setted by pushing anyine of said indicated keies.

BRIEF DESCRIPTION OF THE DRAWINGS

Further features and advantages of the present invention will be apparent from the ensuring description with reference to the accompanying drawings, wherein:

Fig. 1 is a drawing of the first embodiment of the first present invention;

Fig. 2 is a drawing of the second embodiment of the first present invention;

Fig. 3 is a drawing of the third embodiment of the first present invention;

Fig. 4 is a drawing of the first the embodiment of the second present invention;

Fig. 5 is a drawing of the second embodiment of the second present invention; and

Fig. 6 is a drawing of the third embodiment of the second present invention.

## DESCRIPTION OF THE PRIFERRED EMBODIMENTS

(1) First present invention

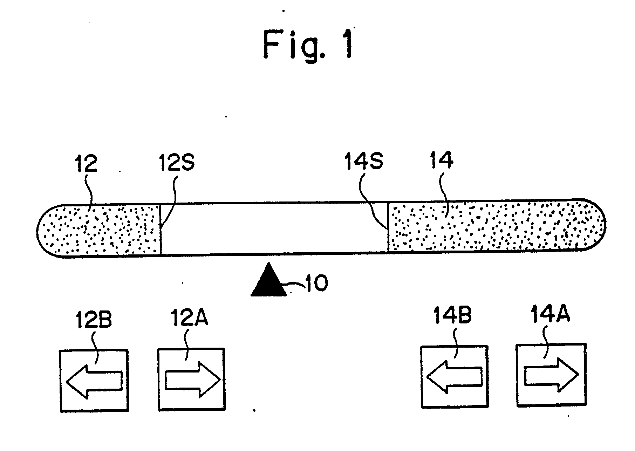

Figure 1 is a drawing of the first embodiment of the first present invention.

In Fig 1, reference numeral 10 shows the present time value of the living body signal of one patient, 14 the upper limit, 12 the lower limit, 14S the threshold of the upper limit, 12S the threshold of the lower limit.

Under the present time value 10, a key 14A for increasing the upper limit 14, a key 14B for decreasing the upper limit 14, a key 12A for increasing the lower limit 12, and a key 12B for decreasing the lower limit 12, are respectively indicated.

When the key 14A is kept to be pushed, the threshold 14S moves to the right looking into the drawing, that is to say, it is kept to increase.

When hands are lost from the key 14A kept to be pushed, the threshold 14S stope to move, whereby the operation of setting for the threshold 14S is ended.

When the key 14B is kept to be pushed, the threshold 14S moves to the left looking into the drawing, that is to say, it is kept to decrease.

When hands are lost from the key 14B kept to be pushed, the threshold 14S stops to move, whereby the operation of setting for the threshold 14S is ended.

Since the operations of the keys 12A and 12B are the same as those of the keys 14A and 14B, the explanation thereof will be omitted.

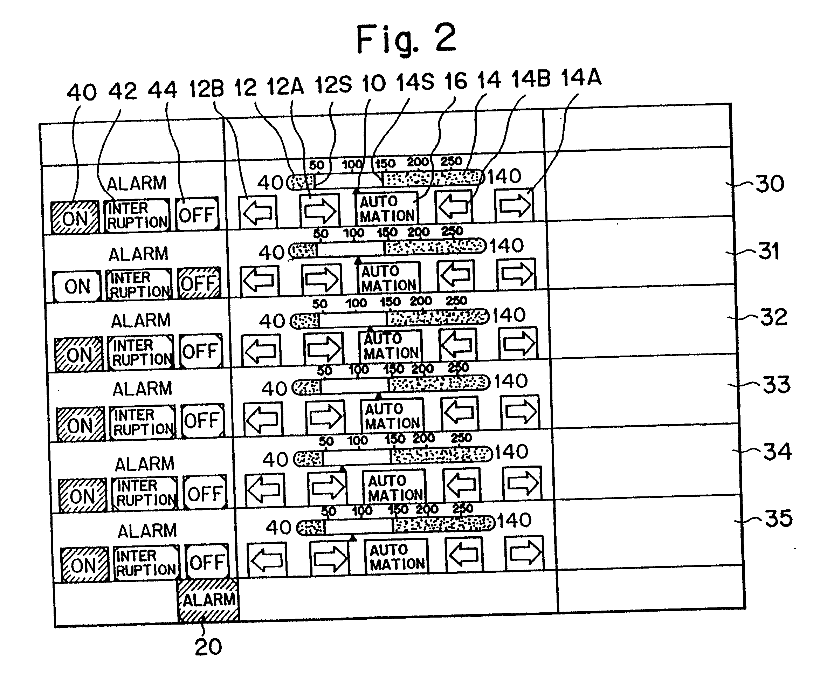

Figure 2 is a drawing of the second embodiment of the first present invention.

In Fig 2, an embodiment is disclosed so as to carry out simultaneously in all the beds the operation of setting alarm, regarding not one patiene but all the patients.

A picture shown in Fig. 2 is adopted to appear by pushing an alarm key 20 indicated on the lower section of the basic picture.

The basic picture is indicated, when the condition of the patient is normally monitorred.

In the basic picture, the living body signal of each patient, for example the wave of electrocardiogram signal, is each indicated in the whole picture.

However, when the alarm key 20 is pused, the wave of electrocardiogram is respectively indicated in compression on the right side regions 30, 31, 32, 33, 34 and 35, as shown in Fig.2.

In Fig.2, the present time valve 10 of the living body signal of each patient is heart rate respectively.

In the picture of Fig.2, scales 50, 100 • • • are indicated, as well as a numerical value 140 of the content of the present time upper limit threshold 14S is indicated on the upside of the key 14A, and a numerical value 40 of the content of the present time lower limit threshold 12S is indicated on the upside of the key 12B.

The operation of setting alarm in accordance with the present invention will be explained herein after.

When the key 14A is kept to be pushed at the same time of looking at the scale, the threshold 14S moves to the right lookng in to the drawing, that is to say, it is kept to increase.

When hands are lost from the key 14A kept to be pushed, at the same time that the threshold 14S is reached the required scale, the threshold 14S stopes to move, whereby the operation of setting for the threshold 14S is ended.

When the key 14B is kept to be pushed, the threshold 14S moves to the left looking into the drawing, that is to say, it is kept to decrease.

When hands are lost from the key 14B kept to be pushed, the threshold 14S stops to move, where by the operation of setting for the threshold 14S is ended.

The operations of the keys 12A and 12B are the same as those of the keys 14A and 14B, as aforementioned.

A key, of reference numeral 16, which is indicated respectively on the central portion of the picture, is a key for setting automatically the upper limit or lower limit.

When the key 16 is pushed, alarm may be setted automatically so that the threshold 14S is +x% or the threshold 12S is -y%, with respect to the present time value 10.

On the left side of the picture, a key 40 for swinching on alarm, a key 42 for interruping alarm, and a key 44 for switching off alarm are indicated respectively, each patient.

When the key 40 is pushed, the above alarm indication is indicated each patient.

When the key 42 is pushed, the above alarm indication is not indicated for the required time.

When the key 44 is pushed, the above alarm indication is not indicated entirely.

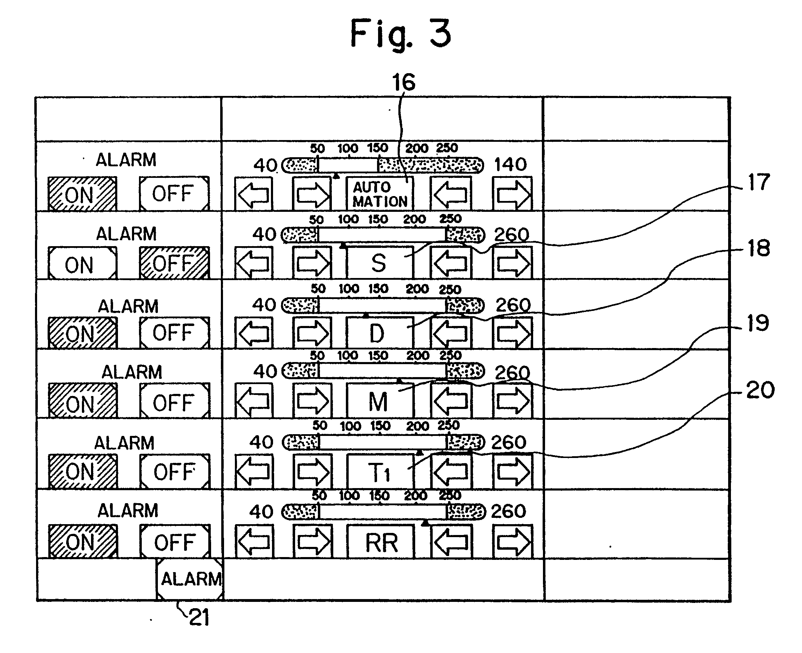

Figure 3 is a drawing of the third embodiment of the first present invention.

The embodiment of Fig. 3 is the case wherein the operation of setting alarm is carried out simultanepusly, with respect to all the parameters of one patient, that is to say, all the living body signals of heart rate, blood pressure, and bodily temperature etc..

In Fig. 3, for example, the operation of setting alarm may be carried out simultaneously, in order from top to bottom, with respect to the parameters of heart rate, blood pressure 1, blood pressure 2, blood pressure 3, bodily temperature, and respiration number.

Since the operation of setting alarm in the embodiment of Fig. 3, is almost the same as that of Fig.2, the different points will be only described herein after.

With respect to the first heart rate, alarm may be setted automatically by pushing a key 16.

With respect to the next blood pressure 1 through the final number of respiration, setting item may be also verified each parameter, alarm can be setted regarding the setting item.

For example, with respect to the blood pressure 1, when a key 17 is kept to be pushed, setting item is verified so that S (for meaning contraction), D (for meaning extension), and M (for meaning average) appear in order.

Regarding each setting item verified, alarm may be setted by the same operation as the embodiment of Fig. 2.

With respect to the blood pressure 2, blood pressure 3, bodily temperature, respiration number, alarm can be setted by the same as that of the blood pressure 1.

As aforementioned, according to the first present invention, a method for setting alarm is provided, where in the present time value, the upper limit and lower limit of the living body signal are indicated, and the operation of setting the threshold of the upper limit or lower limit is carried out by pushing key indicated.

There by, since the present time value, upper limit, and lower limit of the living body signal are indicated, the upper limit or lower limit is adopted to be setted by looking at the above indication.

Therefore, the first present invention has an effect that the operation of setting alarm can be carried out very easily.

Since there is no volume, the first present invention has another effect that the place is not occupied an that the cost of the whole apparatus is low.

(2)Second present invention

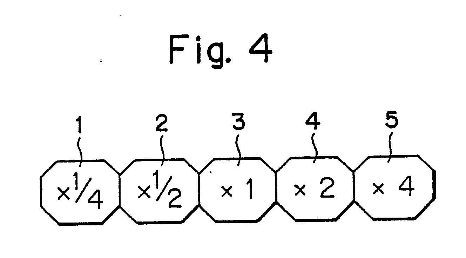

Figure 4 is a drawing of the first embodiment of the second present invention, wherein refereuce numerals 1 to 5 show key for setting wave indicating sensitivity, respectively.

The embodiment of Fig. 4 is the case where wave indicating sensitivity of one living body signal of one patient, f or example electrocardiogram signal, is setted.

The keies 1, 2, 3, 4, and 5 are those used in the case where wave indicating sensitivities are setted to 1/4, 1/2, 1, 2, and 4, respectively.

According to the second present invention, when anyone of the above indicated keies 1 to 5 is pushed, wave indicating sensitivity may be setted with once operation.

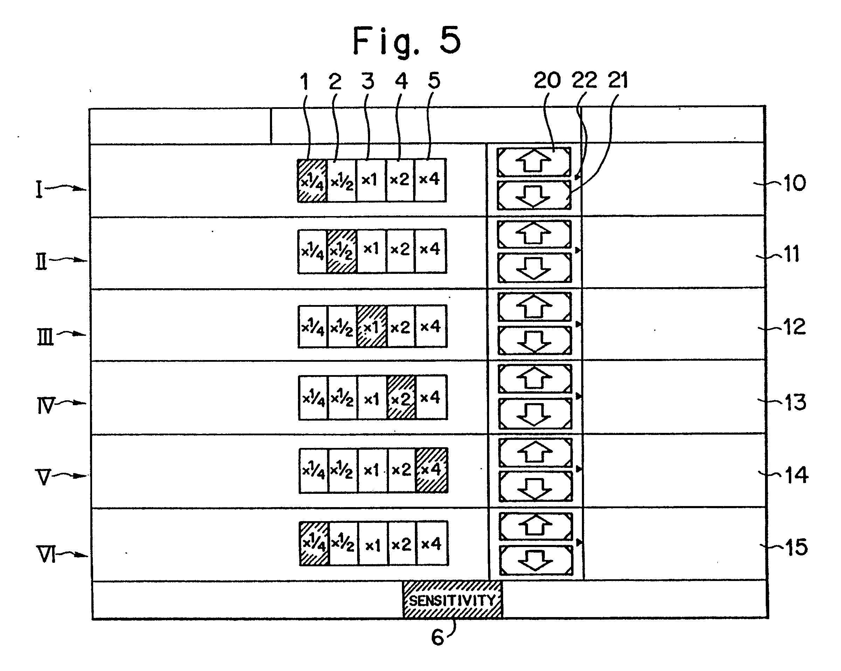

Figure 5 is a drawing of the second embodiment of the second present invention, which is the case where wave indicating sensitivity is setted simultaneously, regarding all the beds.

In Fig. 5, the keies 1 to 5 are shown each beds I, II, III • • • VI.

In regions 10,11 • • • 15 of the right side of the keies 1 to 5, the wave objected to sensitivety, for example, the wave of electrocardiogram signal are indicated, respectively.

Each bed, when the keies 1, 2, 3, 4, and 5 are pused, the sensitivities of the waves become to 1/4, 1/2, 1, 2, and 4, respectively.

By pushing the wave of the basic picture, wave indicating sensitivities may be setted in order to the one directi on, from small sensitivity to large one or from large one to small one.

Alternately, they may be in order cyclically, from small sensitivity to large one or from the large one to small one.

In Fig. 5, keies 20 and 21 for setting the position of the wave are indicated besides keies 1 to 5 and the wave in the regions 10 to 15.

The keies 20 and 21 are those for rising and falling respectively the position of the wave in the regions 10 to 15.

That is to say, when the key 20 is pushed, the wave is adopted to rise, and when the key 21 is pushed, the wave is adopted, to fall.

Reference numeral 22 shows the present time poition of the wave.

The operation of the second present invention will be described herein after, based on Fig. 5.

First, in the basic picture, a sensitivity key 6 is pushed.

Thereby, the keies 1 to 5 are indicated simultaneously in all the beds I to VI, and the waves of electrocardiogram are indicated in the regions 10 to 15, as shewn in Fig. 5.

With respect to the beds I, as shown in obligue lines, when the key 1 is pused, wave indicating sensitivity becomes to 1/4.

With respect to the bed II to VI, when keies 2, 3, 4, 5 and 1 are pused, wave indicating sensitivities become to 1/2, 1 2, 4 and 1/4, respectively.

Therefore, when the sensitivity key 6 is only pushed, a plurality of keies 1 to 5 are indicated simultaneously in all the beds, and when enyone of the keies 1 to 5 are pushed, wave indicating sensitivity may be setted.

Next, the operation of the keies 20 and 21 will be described herein after.

For example, with respect to the bed I, the present time wave position 22 appears as shown in Fig. 5.

Hence, if the whole wave will be fallen, the key 21 may be pushed.

On the contrary, if the whole wave will be risen, the key 20 may be pushed.

Owing to the keies 20 and 21, the wave in setted sensitivity can be observed very easily.

With respect to the other beds II, III • • • VI, the operations of wave position setting keies are the same as the bed I.

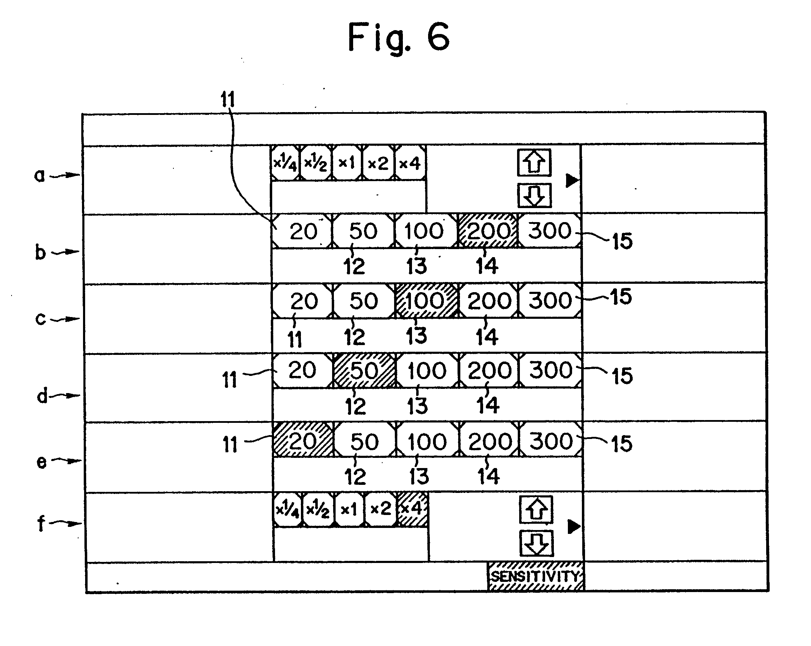

Figure 6 is a drawing of the third embodiment of the second present invention.

The embodiment of Fig. 6 is the case where wave indicating sensitivity is setted simultaneously regarding all the paramenters of one patient, that is to say, the living body signals of edectrocardiogram, blood pressure and bodily temperature etc..

In Fig. 6, for example, with respect to the parameters of the electrocardiogram shown in reference numeral a, the blood pressure 1 shown in b, the blood pressure 2 shown in c, the blood pressure 3 shown in d, the blood pressure 4 shown in e, and the respiration number shown in f, ware indicating sensitivity may be setted simultaneously.

The operation of setting senisitivity of the first electrocardiogram a and the final respiration number f is the same as that of Fig. 5.

On the other hand, the operation of setting sensitivity of the blood pressure 1 to 4 is as follows.

That is to say, when keies 14, 13, 12, and 11 are pushed as shown in obligue lines, the sensitivities of 200mmHg, 100 mmHg, 50mmHg, and 20mmHg may be setted respectively with once operation.

As aforementioned, according to the second present invention, a method for setting wave indicating sensitivity is provided, wherein a plurality of keies for setting sensitivity are indicated, and ware indicating sesitivity is setted by anyone of the above indicated keies.

Hence, when anyone of a plurality of keies for setting sensitivity indicated is pushed, wave indicating sensitivity may be setted with once operation.

Therefore, the second present invention has an effect that the operation of setting sensitivity can be carried out very easily.

**Claims**

1. A method for setting alarm comprising: the present time value, the upper limit and lower limit of the living body signal being indicated, the operation of setting the threshold of the upper limit or lower limit carried out by pushing keies indicated.

2. A method for setting alarm according to claim 1, wherein said indication may be carried out simultaneously in all the beds.

3. A method for setting alarm according to claim 1, wherein said indication may be carried out simultaneously regarding all the parameters.

4. A method for setting alarm according to claim 1, wherein said keies are a key for increasing the upper limit, a key for decreasing the upper limit, a key for increasing the lower limit, and a key for decreasing the lower limit.

5. A method for setting alarm according to claim 1, wherein said operation of setting the threshold may be carried out automatically by a key for setting automatically the upper limit or the lower limit.

6. A method for setting alarm according to claim 1, wherein said indication may be switched on or switched off.

7. A method for setting alarm according to claim 1, wherein said indication may be interrupted for the reguired time.

8. A method for setting wave indicating sensitivity comprising: a plurality of keies for setting sensitivity indicated, wave indication sensitivity being setted by pushing anyone of said indicated keies.

9. A method for setting wave indicating sensitivity according to claim 8, wherein said indication may be carried out simultaneously in all the beds.

10. A method for setting wave indicating sensitivity according to claim 8, wherein said indication may be carried out simultaneously regarding all the parameters.

11. A method for setting wave indicating sensitivity according to claim 8, wherein the position of the wave may be setted besides said wave indication sensitivity.

12. A method for setting wave indicating sensitivity according to claim 8, wherein said wave indication sensitivity may be setted in order to one direction, from small sensitivity to large one or from large one to small one, by pushing the wave indicated.

13. A method for setting wave indicating sensitivity accordining to claim 8,
wherein said wave indication sensitivity may be setted in order cyclically, from small sensitivity to large one and from the large one to the small one, by pushing the wave indicated.

## Fig. 1

# Fig. 2

40 42 44 12B 12 12A 12S 10 14S 16 14 14B 14A

|  |  |  |  |
|---|---|---|---|
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～30 |
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～31 |
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～32 |
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～33 |
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～34 |
| ALARM <br> ON INTER RUPTION OFF | 50 100 150 200 250 <br> 40 ⊂▭⊃ ▭ 140 <br> ⇦ ⇨ AUTO MATION ⇦ ⇨ |  | ～35 |
| ALARM |  |  |  |

20

EP 0 384 960 A2

Fig. 3

# Fig. 4

# Fig. 5

EP 0 384 960 A2

# Fig. 6

EP 0 384 960 A2